(19) Europäisches Patentamt
European Patent Office
Office européen des brevets

(11) Numéro de publication : **0 390 681 B1**

# (12) FASCICULE DE BREVET EUROPEEN

(45) Date de publication du fascicule du brevet : **25.08.93 Bulletin 93/34**

(51) Int. Cl.$^5$ : **C07C 49/747,** C07C 49/753, A61K 7/40, A61K 31/125

(21) Numéro de dépôt : **90400846.3**

(22) Date de dépôt : **28.03.90**

## (54) Compositions cosmétiques et pharmaceutiques contenant des dérivés lipophiles du benzylidène camphre et nouveaux dérivés lipophiles du benzylidène camphre.

(30) Priorité : **31.03.89 FR 8904297**

(43) Date de publication de la demande : **03.10.90 Bulletin 90/40**

(45) Mention de la délivrance du brevet : **25.08.93 Bulletin 93/34**

(84) Etats contractants désignés : **AT BE CH DE DK FR GB IT LI NL SE**

(56) Documents cités :
**FR-A- 2 395 023**
**GB-A- 2 025 957**

(73) Titulaire : **L'OREAL**
**14, Rue Royale**
**F-75008 Paris (FR)**

(72) Inventeur : **Forestier, Serge**
**16 Allée Ferdinand Buisson**
**F-77410 Claye-Souilly (FR)**
Inventeur : **Lagrange, Alain**
**29, rue Auguste Renoir**
**F-78400 Chatou (FR)**
Inventeur : **Lang, Gérard**
**44 avenue Lacour**
**F-95210 Saint-Gratien (FR)**
Inventeur : **Deflandre, André**
**Route de Manon**
**F-60560 Orry-la-Ville (FR)**
Inventeur : **Luppi, Bernadette**
**43, rue Berlioz**
**F-93270 Sevran (FR)**

(74) Mandataire : **Casalonga, Axel et al**
**BUREAU D.A. CASALONGA - JOSSE**
**Morassistrasse 8**
**D-80469 München (DE)**

EP 0 390 681 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet des compositions cosmétiques à usage quotidien ou antisolaire et des compositions pharmaceutiques pour le traitement des inflammations et allergies cutanées contenant des dérivés lipophiles du benzylidène camphre ainsi que de nouveaux dérivés lipophiles du benzylidène camphre.

Il est bien connu que la peau est sensible aux radiations solaires qui peuvent provoquer un banal coup de soleil ou érythème mais aussi des brûlures plus ou moins accentuées.

Cependant, les radiations solaires ont également d'autres effets néfastes tels qu'une perte d'élasticité de la peau et l'apparition de rides conduisant à un vieillissement prématuré. On peut même aussi observer parfois des dermatoses.

Il est également souhaitable d'assurer aux cheveux une bonne protection contre la dégradation photochimique afin d'éviter un changement de nuance, une décoloration ou une dégradation des propriétés mécaniques.

On sait par ailleurs que les constituants entrant dans les préparations cosmétiques ne possèdent pas toujours une stabilité suffisante à la lumière et se dégradent sous l'action des radiations lumineuses.

Il est bien connu que la partie la plus dangereuse du rayonnement solaire est constituée par les radiations ultraviolettes de longueurs d'onde inférieures à 400 nm. On sait aussi que, de par l'existence de la couche d'ozone de l'atmosphère terrestre qui absorbe une partie du rayonnement solaire, la limite inférieure du rayonnement ultraviolet atteignant la surface de la terre se situe aux environs de 280 nm.

Par conséquent, il paraît souhaitable de disposer de composés susceptibles d'absorber les radiations ultraviolettes dans une large bande de longueurs d'onde allant de 280 à 400 nm, c'est-à-dire aussi bien les rayons UV-B de longueurs d'onde comprises entre 280 et 320 nm jouant un rôle prépondérant dans la production de l'érythème solaire, que les rayons UV-A de longueurs d'onde comprises entre 320 et 400 nm provoquant le brunissement de la peau, mais aussi son vieillissement et favorisant le déclenchement de la réaction érythémateuse ou amplifiant cette réaction chez certains sujets ou pouvant même être à l'origine de réactions phototoxiques ou photoallergiques.

La demande de brevet GB-A-2 025 957 décrit, en tant que filtres solaires à bande large, la 4'-hydroxybenzylidène 2-bornanone ainsi que des 3',4'-dialcoxybenzylidène 2-bornanones pouvant être préparées à partir de 4'-hydroxy-3'-alcoxybenzylidène 2-bornanones.

Au cours de ses recherches, la demanderesse vient de découvrir que les dérivés lipophiles du benzylidène camphre ayant la formule suivante :

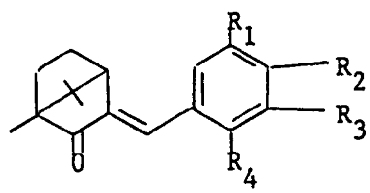

(I)

dans laquelle :

$R_1$ représente un atome d'hydrogène, un radical hydroxyle, un radical alkyle linéaire ou ramifié en $C_1$-$C_3$ ou un radical alcoxy linéaire ou ramifié en $C_1$-$C_8$,

$R_3$ représente un radical hydroxyle, un radical alkyle linéaire ou ramifié en $C_1$-$C_3$ ou un radical alcoxy linéaire ou ramifié en $C_1$-$C_8$, ou bien lorsque $R_1$ représente un atome d'hydrogène ou un radical hydroxyle, $R_3$ peut représenter un reste alkyle linéaire ou ramifié en $C_1$-$C_8$,

$R_2$ et $R_4$, identiques ou différents, représentent un atome d'hydrogène ou un radical hydroxyle, étant entendu que l'un au moins des radicaux $R_2$ et $R_4$ représente un radical hydroxyle,

présentaient, de manière inattendue, outre de bonnes propriétés filtrantes dans la gamme de longueurs d'onde allant de 280 à 380 nm, d'excellentes propriétés anti-oxydantes vis-à-vis de la peroxydation des lipides polyinsaturés et également vis-à-vis des substances susceptibles de subir des réactions d'oxydation thermo- ou photo-induites (telles que des protéines, des sucres, des pigments, des vitamines, des polymères...).

Or, on sait que la peroxydation des lipides implique la formation de radicaux libres intermédiaires qui endommagent les membranes cellulaires se composant, entre autres, de phospholipides et sont responsables notamment des phénomènes de vieillissement de la peau (A.L TAPPEL dans "Federation Proceedings" Vol. 32, No 8, Août 1973).

Il est donc extrêmement intéressant de disposer de composés présentant à la fois des propriétés filtrantes dans une large bande et des propriétés anti-oxydantes potentialisant l'effet filtre. De tels composés peuvent

permettre par exemple de mieux lutter contre le vieillissement prématuré de la peau dû à la peroxydation des lipides cutanés.

De tels composés peuvent aussi permettre d'assurer une meilleure conservation des compositions cosmétiques ou pharmaceutiques comportant une phase grasse en évitant le rancissement des lipides insaturés qui y sont contenus et qui peuvent être d'origine animale comme la lanoline, la cétine (blanc de baleine), la cire d'abeille, le perhydrosqualène, l'huile de tortue, ou végétale comme l'huile d'olive, l'huile de ricin, l'huile de maïs, l'huile d'amande douce, l'huile d'avocat, l'huile de karité, l'huile de tournesol, l'huile de soja, l'huile d'arachide, les huiles de coprah ou de palmiste, des acides gras essentiels comme la vitamine F et certaines huiles essentielles présentes dans les parfums comme l'huile de citron ou de lavande.

Ils peuvent également permettre d'éviter la dégradation oxydative de composés actifs contenus dans les compositions pharmaceutiques (vitamine A, caroténoïdes...)

La demanderesse a également découvert de manière extrêmement surprenante, que les composés de formule (I) ci-dessus pouvaient être utilisés pour le traitement des inflammations et allergies cutanées.

Outre leurs bonnes propriétés filtrantes et anti-oxydantes, les composés (I) présentent un excellent caractère lipophile ainsi qu'une très bonne stabilité thermique et photochimique.

Ces composés présentent également l'avantage de ne pas être toxiques ou irritants et d'avoir une parfaite innocuité vis-à-vis de la peau.

Ils se répartissent uniformément dans des supports cosmétiques classiques aptes à former un film continu et notamment dans les supports gras et peuvent ainsi être appliqués sur la peau pour constituer un film protecteur efficace.

Certains des composés de formule (I) ci-dessus sont nouveaux.

Il s'agit des dérivés lipophiles du benzylidène camphre de formule (I') suivante :

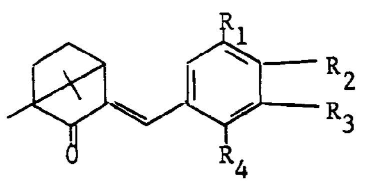

(I')

dans laquelle $R_1$ à $R_4$ ont les significations indiquées pour la formule (I), sous réserve que lorsque $R_1$ et $R_4$ représentent un atome d'hydrogène et $R_2$ un radical hydroxyle, $R_3$ ne représente pas un radical alcoxy.

Dans la formule (I'), $R_1$ et $R_3$ peuvent désigner par exemple un radical méthyle, éthyle, n-propyle, isopropyle, n-butyle, isobutyle ou tertiobutyle, ou bien un reste méthoxy, éthoxy, propoxy, butoxy, pentyloxy, hexyloxy, heptyloxy ou octyloxy.

Parmi les composés préférés de formule (I'), on peut citer :

- le 3'-tert.-butyl-4'-hydroxy 3-benzylidène camphre,
- le 3',5'-diméthyl-4'-hydroxy-3-benzylidène camphre,
- le 3',5'-diisopropyl-4'-hydroxy-3-benzylidène camphre,
- le 3',5'-diméthoxy-4'-hydroxy-3-benzylidène camphre,
- le 3',4',5'-trihydroxy-3-benzylidène camphre,

La présente invention a donc pour objet les composés de formule (I') ci-dessus.

Les composés de formule (I) ou (I') sont obtenus à partir du camphre synthétique (d,1-camphre) ou à partir du camphre naturel (d-camphre) par condensation avec un aldéhyde aromatique de formule (II) selon le schéma réactionnel suivant :

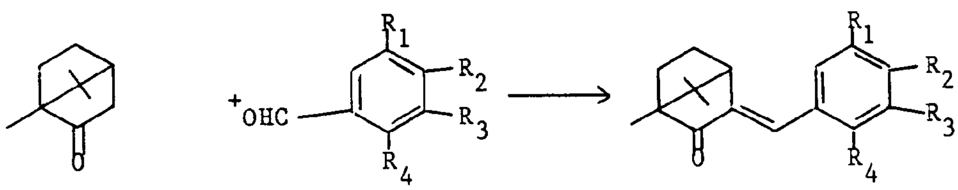

(II) (I) ou (I')

Les aldéhydes de formule (II) dans lesquels les substituants $R_1$ à $R_4$ ont les significations mentionnées ci-dessus pour les composés de formule (I) ou (I') sont des composés connus.

La condensation de l'aldéhyde (II) sur le camphre peut être effectuée en présence d'un alcoolate de métal

alcalin tel que le méthylate de sodium ou le tertiobutylate de potassium, dans un solvant tel que le toluène, le 1,2-diméthoxyéthane ou le 1,2-diéthoxyéthane à une température comprise entre -78°C et le point d'ébullition du solvant. La condensation peut également être effectuée en présence d'une base minérale telle qu'un amidure ou un hydrure de métal alcalin, en présence d'un solvant tel que le 1,2-diméthoxyéthane, ou un hydroxyde de métal alcalin dans le toluène ou un alcool, à une température comprise entre 0°C et le point d'ébullition du mélange réactionnel.

Selon une variante, on peut utiliser un aldéhyde de formule II dont la (ou les) fonction(s) hydroxyle est (ou sont) bloquée(s) par un groupement protecteur tel que benzyle, triméthylsilyle ou tert.-butyldiméthylsilyle, la condensation avec le camphre s'effectuant dans les mêmes conditions que ci-dessus.

Selon cette variante, la réaction de condensation est suivie d'une hydrogénolyse du dérivé protégé pour former le dérivé hydroxylé. L'hydrogénolyse du groupe benzyle peut consister soit en une hydrogénation catalytique, soit en une réduction en présence d'un agent de transfert d'hydrogène, par exemple au moyen de cyclohexène ou d'acide formique, en présence d'un catalyseur tel que le palladium sur charbon, éventuellement en présence d'un solvant inerte.

L'hydrogénolyse des groupements silylés s'effectue, de façon connue, en présence d'ions fluorure.

Un autre objet de l'invention est une composition cosmétique comprenant, dans un support cosmétiquement acceptable, une quantité efficace d'au moins un dérivé de benzylidène camphre de formule (I') ci-dessus, à titre d'agent anti-oxydant et filtrant les rayons UV de longueurs d'onde comprises entre 280 et 380 nm.

La composition cosmétique de l'invention peut être utilisée comme composition protectrice de l'épiderme humain ou des cheveux ou comme composition anti-solaire.

La présente invention a également pour objet un procédé de protection de la peau et des cheveux naturels ou sensibilisés vis-à-vis du rayonnement solaire, consistant à appliquer sur la peau ou les cheveux une quantité efficace d'au moins un composé de formule (I') contenu dans un support cosmétiquement acceptable.

On entend par "cheveux sensibilisés" des cheveux ayant subi un traitement de permanente, de coloration ou de décoloration.

L'invention a également pour objet une composition cosmétique colorée ou non colorée, stabilisée à la lumière et/ou à l'oxydation, comprenant une quantité efficace d'au moins un dérivé de benzylidène camphre de formule (I') ci-dessus.

Lorsqu'elle est utilisée comme composition destinée à protéger l'épiderme humain contre les rayons ultraviolets ou comme composition antisolaire, la composition cosmétique selon l'invention peut se présenter sous les formes les plus diverses habituellement utilisées pour ce type de composition. Elle peut notamment se présenter sous forme d'émulsions telles qu'une crème ou un lait, de lotions huileuses, alcooliques ou hydroalcooliques, de gels oléoalcooliques ou hydroalcooliques, de bâtonnets solides, ou être conditionnée en aérosol pour former une mousse ou un spray.

Elle peut contenir les adjuvants cosmétiques habituellement utilisés dans ce type de composition tels que des épaississants, des adoucissants, des humectants, des tensio-actifs, des conservateurs, des anti-mousses, des parfums, des huiles, des cires, de la lanoline, des propulseurs, des colorants et/ou pigments ayant pour fonction de colorer la composition elle-même ou la peau ou tout autre ingrédient habituellement utilisé en cosmétique.

Le composé de formule (I') est présent dans des proportions en poids comprises entre 0,1 et 4% par rapport au poids total de la composition cosmétique protectrice de l'épiderme humain.

Comme solvant de solubilisation, on peut utiliser une huile, une cire et de façon générale tout corps gras, un monoalcool ou un polyol inférieur ou leurs mélanges. Les monoalcools ou polyols plus particulièrement préférés sont l'éthanol, l'isopropanol, le propylèneglycol, la glycérine et le sorbitol.

Une forme de réalisation de l'invention est une émulsion sous forme de crème ou de lait protecteurs comprenant en plus du composé de formule (I'), des alcools gras, des esters d'acides gras et notamment des triglycérides d'acides gras, des acides gras, de la lanoline, des huiles ou cires naturelles ou synthétiques et des émulsionnants, en présence d'eau.

Une autre forme de réalisation est constituée par des lotions huileuses à base d'huiles et cires naturelles ou synthétiques, de lanoline et d'esters d'acides gras, notamment de triglycérides d'acides gras, ou par des lotions oléoalcooliques ou hydroalcooliques à base d'un alcool inférieur tel que l'éthanol ou d'un glycol tel que le propylèneglycol, et/ou d'un polyol tel que la glycérine et d'eau ou bien d'huiles, de cires et d'esters d'acides gras tels que les triglycérides d'acides gras.

La composition cosmétique de l'invention peut également être un gel oléoalcoolique ou hydroalcoolique comprenant un ou plusieurs alcools ou polyols inférieurs tels que l'éthanol, le propylèneglycol ou la glycérine et un épaississant. Les gels oléoalcooliques contiennent une huile ou une cire naturelle ou synthétique.

Les bâtonnets solides sont constitués de cires et d'huiles naturelles ou synthétiques, d'alcools gras, d'esters d'acides gras, de lanoline et autres corps gras.

La présente invention vise également les compositions cosmétiques anti-solaires contenant au moins un composé de formule (I') et pouvant contenir d'autres filtres UV-B et/ou UV-A.

Dans ce cas, la quantité de filtre de formule (I') est comprise entre 0,2 et 15% en poids, la quantité totale de filtres présents dans la composition anti-solaire, c'est-à-dire le composé de formule (I') et éventuellement les autres filtres, étant comprise entre 0,5 et 15% en poids par rapport au poids total de la composition anti-solaire.

Lorsque la composition cosmétique selon l'invention est destinée à protéger des rayons UV les cheveux naturels ou sensibilisés, cette composition peut se présenter sous forme de shampooing, de lotion, gel ou émulsion à rincer, à appliquer avant ou après le shampooing, avant ou après coloration ou décoloration, avant ou après permanente, de lotion ou gel coiffants ou traitants, de lotion ou gel pour le brushing ou la mise en plis, de laque pour cheveux, de composition de permanente, de coloration ou décoloration des cheveux. Cette composition peut contenir, outre le composé de l'invention, divers adjuvants utilisés dans ce type de composition, tels que des agents tensio-actifs, des épaississants, des polymères, des adoucissants, des conservateurs, des stabilisateurs de mousses, des électrolytes, des solvants organiques, des dérivés siliconés, des huiles, des cires, des agents anti-gras, des colorants et/ou pigments ayant pour fonction de colorer la composition elle-même ou la chevelure ou tout autre ingrédient habituellement utilisé dans le domaine capillaire.

Elle contient 0,25 à 4% en poids de composé de formule (I').

La présente invention vise également les compositions cosmétiques renfermant un constituant sensible à la lumière et/ou à l'oxydation et contenant au moins un composé de formule (I') à titre d'agent de protection contre les rayons ultraviolets et agent anti-oxydant. Ces compositions sont constituées par des compositions capillaires telles que les laques pour cheveux, les lotions de mise en plis éventuellement traitantes ou démêlantes, les shampooings, les shampooings colorants, les compositions tinctoriales pour cheveux, par des produits de maquillage tels que les vernis à ongles, les crèmes et huiles de traitement pour l'épiderme, les fonds de teint, les bâtons de rouge à lèvre, les compositions pour les soins de la peau telles que des huiles ou crèmes pour le bain, ainsi que toute autre composition cosmétique pouvant présenter du fait de ses constituants, des problèmes de stabilité à la lumière et/ou à l'oxydation au cours du stockage.

De telles compositions contiennent 0,1 à 4% en poids de composé de formule (I').

L'invention vise également un procédé de protection des compositions cosmétiques ou pharmaceutiques contre les rayons ultra-violets et l'oxydation, consistant à incorporer à ces compositions une quantité efficace d'au moins un composé de formule (I').

Un autre objet de l'invention est l'utilisation des composés de formule (I') en tant que filtres solaires à large bande absorbant dans la gamme de longueurs d'onde allant de 280 à 380 nm.

L'invention a également pour objet l'application à titre de produits cosmétiques des composés de formule (I').

Un autre objet de l'invention est l'utilisation en tant qu'agents anti-oxydants des composés de formule (I).

L'effet anti-oxydant de ces composés peut être mis en évidence par chimiluminescence d'homogénats cellulaires. Cette technique est basée sur l'émission spontanée de lumière qui résulte de la désactivation radiative des produits de décomposition des lipides peroxydés. L'étude cinétique s'effectue sur des broyats de cervelles de rats, dilués dans un tampon phosphate, et est menée parallèlement avec une solution témoin ne contenant pas d'anti-oxydant et des solutions contenant l'antioxydant à différentes concentrations variant de $10^{-7}$ à $10^{-5}$ molaire.

Par référence au témoin, on peut déterminer les concentrations Cx en anti-oxydant permettant l'inhibition de x % de la peroxydation.

Comme on l'a indiqué ci-dessus, la demanderesse a en outre découvert au cours de ses recherches, que les composés de formule (I) présentaient une activité pharmacologique intéressante dans le domaine du traitement des inflammations et allergies cutanées.

L'invention a donc pour objet le composé de formule (I) pour son utilisation comme médicament.

L'invention a également pour objet une composition pharmaceutique contenant une quantité efficace d'au moins un composé de formule (I) à titre d'ingrédient actif, dans un support ou un excipient non toxique.

A titre de composés (I) préférés, on peut citer :

- le 3'-tert.-butyl-4'-hydroxy-3-benzylidène camphre,
- le 3'-méthoxy-4'-hydroxy-3-benzylidène camphre,
- le 3'-éthoxy-4'-hydroxy-3-benzylidène camphre,
- le 3',5'-diméthyl-4'-hydroxy-3-benzylidène camphre
- le 3',5'-diisopropyl-4'-hydroxy-3-benzylidène camphre,
- le 3',5'-diméthoxy-4'-hydroxy-3-benzylidène camphre,
- le 3',4',5'-trihydroxy-3-benzylidène camphre.

La composition pharmaceutique conforme à l'invention peut être administrée par voie orale ou par voie

topique.

Pour la voie orale, la composition pharmaceutique peut se présenter sous forme de comprimés, de gélules, de dragées, de sirops, de suspensions, de solutions, d'émulsions etc.. Pour la voie topique, la composition pharmaceutique selon l'invention se présente sous forme d'onguent, de crème, de pommade, de solution, de lotion, de gel, spray, suspension, etc.

Cette composition médicamenteuse peut contenir des additifs inertes ou pharmacodynamiquement actifs et notamment : des agents hydratants, des antibiotiques, des agents anti-inflammatoires stéroïdiens ou non stéroïdiens, des caroténoïdes, des agents anti-psoriasiques.

Cette composition peut également contenir des agents d'amélioration de la saveur, des agents conservateurs, des agents stabilisants, des agents régulateurs d'humidité, des agents régulateurs de pH, des agents modificateurs de la pression osmotique, des agents émulsionnants, des anesthésiques locaux, des tampons etc.

Elle peut aussi être conditionnée sous des formes retard ou à libération progressive connues en elles-mêmes.

Le composé de formule (I) conforme à l'invention est présent dans les compositions pharmaceutiques dans des proportions comprises entre 0,01 et 80 % en poids, par rapport au poids total de la composition et de préférence entre 0,1 et 20 % en poids.

Dans l'application thérapeutique, le traitement est déterminé par le médecin et peut varier selon l'âge, le poids et la réponse du patient ainsi que la gravité des symptômes.

Lorsque les composés de formule (I) sont administrés par voie orale, le dosage est généralement compris entre 0,1 et 50 mg/kg/jour et de préférence entre 0,2 et 20 mg/kg/jour. La durée du traitement est variable suivant la gravité des symptômes et peut s'étaler entre 1 et 25 semaines, de façon continue ou discontinue.

Les compositions par voie topique contiennent de préférence de 0,25 % à 4 % en poids de composé de formule (I).

Comme support ou excipient de la composition pharmaceutique de l'invention, on peut utiliser tous supports ou excipients conventionnels non toxiques.

Les exemples suivants sont destinés à illustrer l'invention sans pour autant présenter un caractère limitatif.

## EXEMPLES DE PREPARATION

### EXEMPLE 1

Préparation d'un composé de formule générale (I') dans laquelle $R_1$= $R_4$= H, $R_2$= OH, $R_3$= tert.-butyle :

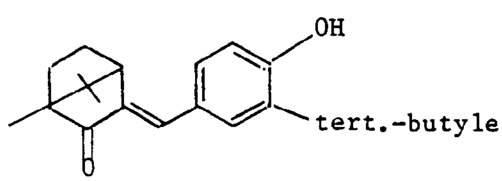

a) Préparation du 4'-benzyloxy-3'-tert.-butyl-3-benzylidène-camphre

On chauffe au reflux pendant 15 minutes 3 g (0,02 mole) de d,1-camphre et 1,1 g (0,02 mole) de méthylate de sodium dans 30 $cm^3$ de 1,2-diméthoxyéthane. On ajoute à chaud une solution de 5,4 g (0,02 mole) de 4-benzyloxy-3-tert.-butyl benzaldéhyde dans 20 $cm^3$ de 1,2-diméthoxyéthane et on maintient au reflux pendant 4 heures.

Le mélange réactionnel est refroidi à température ambiante puis versé dans une solution aqueuse de chlorure de sodium acidifiée par de l'acide chlorhydrique. On extrait au dichlorométhane. Le solvant est distillé sous pression réduite et le résidu est recristallisé dans le minimum de cyclohexane.

On obtient le produit attendu sous forme de cristaux blancs avec un rendement de 45%.

b) Préparation du 3'-tert.-butyl-4'-hydroxy-3-benzylidène camphre

On agite, sous argon, pendant 2 heures 30 minutes à température ambiante, puis pendant 1 heure au bain marie bouillant, 3,2 g (0,008 mole) de 4'-benzyloxy-3'-tert.-butyl-3-benzylidène camphre obtenu ci-dessus et 2 g de palladium sur charbon contenant 50% d'eau (5% en poids de palladium par rapport au charbon) dans 40 $cm^3$ d'acide formique. On laisse refroidir puis on dilue par 20 $cm^3$ d'eau. On filtre sur Célite. Le gâteau est lavé au dichlorométhane puis à l'eau. La phase organique est lavée à l'eau puis évaporée à sec. Le résidu est recristallisé dans l'éther diisopropylique.

On obtient 1 g de produit attendu sous forme de cristaux blancs possédant les caractéristiques suivantes :
- Point de fusion : 216°C
- Analyse élémentaire : $C_{21}H_{28}O_2$

| | C% | H% | 0% |
|---|---|---|---|
| Calculé | 80,73 | 9,03 | 10,24 |
| Trouvé | 80,71 | 9,10 | 10,37 |

- Spectre UV($CH_2Cl_2$)
  . $\lambda_{max}$ : 316 nm
  . $\varepsilon_{max}$ : 23700

EXEMPLE 2

Préparation d'un composé de formule générale (I) dans laquelle $R_1$= $R_4$= H, $R_2$= OH, $R_3$= $OCH_3$ :

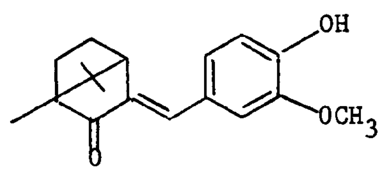

On dissout 192 g (1,26 mole) de d,1-camphre dans 1 litre de 1,2-diméthoxyéthane sec. On ajoute sous azote, 120 g (2,52 moles) d'hydrure de sodium en suspension à 50% dans l'huile, préalablement lavé avec de l'hexane sec. On porte à 80°C pendant 1 heure 30 minutes puis on ajoute, goutte à goutte, en 1 heure 30 minutes, une solution de 183 g (1,2 mole) de vanilline dans 350 $cm^3$ de 1,2-diméthoxyéthane sec. On chauffe le mélange réactionnel au reflux pendant 10 heures. Après refroidissement, on verse lentement sous agitation dans 3 kg de glace. On acidifie par de l'acide chlorhydrique 6N puis on extrait au dichlorométhane. La phase organique est séchée sur sulfate de sodium. Le solvant est distillé sous pression réduite. Le produit est recristallisé deux fois dans l'éther diisopropylique.

On obtient 150 g de produit attendu sous forme de cristaux blancs possédant les caractéristiques suivantes :
- Point de fusion : 118°C
- Analyse élémentaire : $C_{18}H_{22}O_3$

| | C % | H % |
|---|---|---|
| Calculé | 75,49 | 7,74 |
| Trouvé | 75,56 | 7,73 |

- Spectre UV (éthanol)
  . $\lambda_{max}$ : 334 nm
  . $\varepsilon_{max}$ : 19600

EXEMPLE 3

Préparation d'un composé de formule générale (I) dans laquelle $R_1$= $R_4$= H,$R_2$= OH, $R_3$= $OC_2H_5$

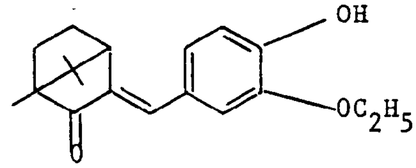

Ce composé est obtenu selon le mode opératoire décrit à l'exemple 2 ci-dessus dans lequel la vanilline est remplacée par l'éthylvanilline.

Le produit obtenu possède les caractéristiques suivantes :

- Point de fusion : 100°C
- Analyse élémentaire : $C_{19}H_{24}O_3$

| | C% | H% | O% |
|---|---|---|---|
| Calculé | 75,97 | 8,05 | 15,98 |
| Trouvé | 76,10 | 8,10 | 16,22 |

Spectre UV (méthanol)

. $\lambda_{max}$ : 335 nm

. $\varepsilon_{max}$ : 19500

EXEMPLE 4

Préparation d'un composé de formule générale (I') dans laquelle $R_1$= $R_3$= $CH_3$, $R_2$= OH, $R_4$= H

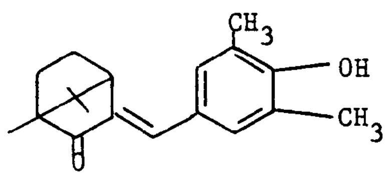

Ce composé est obtenu selon le mode opératoire décrit à l'exemple 2 ci-dessus dans lequel la vanilline est remplacée par le 3',5'-diméthyl-4'-hydroxy benzaldéhyde et l'hydrure de sodium par la quantité équivalente de tert.-butylate de potassium.

Le produit obtenu possède les caractéristiques suivantes :

- Point de fusion : 170°C
- Analyse élémentaire : $C_{19}H_{24}O_2$

| | C % | H % | O % |
|---|---|---|---|
| Calculé | 80,24 | 8,51 | 11,25 |
| Trouvé | 80,21 | 8,50 | 11,28 |

- Spectre UV ($CH_2Cl_2$)

  . $\lambda_{max}$ : 318 nm

  . $\varepsilon_{max}$ : 24100

EXEMPLE 5

Préparation d'un composé de formule générale (I') dans laquelle $R_1$= $R_3$= $OCH_3$, $R_2$= OH,$R_4$= H

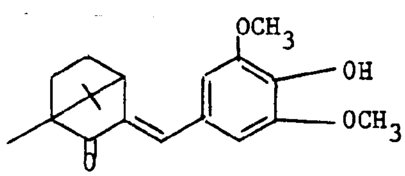

Ce composé est obtenu selon le mode opératoire décrit à l'exemple 2 ci-dessus dans lequel la vanilline est remplacée par le 3',5'-diméthoxy-4'-hydroxybenzaldéhyde et l'hydrure de sodium par la quantité équivalente de tert.-butylate de potassium.

Le produit obtenu possède les caractéristiques suivantes :
- Point de fusion : 158°C
- Analyse élémentaire : $C_{19}H_{24}O_4$

| | C% | H% | 0% |
|---|---|---|---|
| Calculé | 72,13 | 7,65 | 20,23 |
| Trouvé | 72,17 | 7,64 | 20,30 |

- Spectre UV (dichlorométhane)
  . $\lambda_{max}$: 327 nm
  . $\varepsilon_{max}$ : 21300

EXEMPLE 6

Préparation d'un composé de formule générale (I') dans laquelle $R_1$= $R_3$= isopropyle, $R_2$= OH, $R_4$= H :

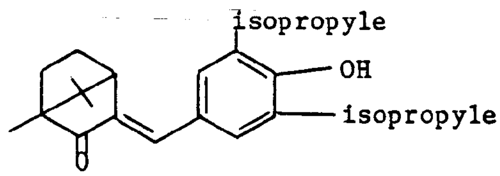

Ce composé est obtenu selon le mode opératoire décrit à l'exemple 2 ci-dessus dans lequel la vanilline est remplacée par le 3',5'-diisopropyl-4'-hydroxy benzaldéhyde et l'hydrure de sodium par la quantité équivalente de tert.-butylate de potassium.

Le produit obtenu possède les caractéristiques suivantes :
- Point de fusion :159-160°C
- Analyse élémentaire : $C_{23}H_{32}O_2$

| | C% | H% | 0% |
|---|---|---|---|
| Calculé | 81,13 | 9,47 | 9,40 |
| Trouvé | 81,08 | 9,41 | 9,66 |

- Spectre UV ($CH_2Cl_2$)
  . $\lambda_{max}$ : 318 nm
  . $\varepsilon_{max}$ : 25 300

Mise en évidence des propriétés anti-oxydantes par chimiluminescence d'homogénats cellulaires

Le test de chimiluminescence est exécuté selon la publication de E.A. LISSI, T. CACERES et L.A. VIDELA "Visible Chemiluminescence from rat brain homogenates undergoing autoxidation. I. Effect of additives and products accumulation" dans "Journal of Free radicals in Biology and Medicine", Vol. 2, pp. 63-69 (1986). Ce test a été simplifié dans le sens qu'on a mesuré uniquement la chimiluminescence sans mesurer l'accumulation de l'acide thiobarbiturique après 1 heure d'irradiation. Cependant, selon la publication, il y a une bonne corrélation entre la chimiluminescence et la mesure de l'accumulation de l'acide thiobarbiturique.

Des cervelles de rats sont prélevées immédiatement après dislocation des cervicales. Elles sont lavées avec une solution tampon contenant 140 mM de NaCl et 40 mM de phosphate de potassium, débarrassées des vaisseaux apparents et de la membrane extérieure, puis broyées avec 4 fois leur volume de solution tampon. Ce broyat est dilué 3 fois avec la solution tampon (dilution totale 12 fois par rapport aux organes de départ).

10 ml d'homogénat dilué est introduit dans des flacons de comptage contenant 200 µl de solution du produit anti-oxydant à tester dissous à différentes concentrations dans du méthanol ou du diméthylsulfoxyde. Immédiatement après agitation, les flacons sont introduits dans un compteur de scintillation (BECKMAN) et comptés périodiquement.

Parallèlement, une étude est menée sur une solution témoin ne contenant pas d'anti-oxydant.

On établit ainsi, pour chaque concentration, une courbe d'intensité de chimiluminescence en fonction du temps.

Par comparaison des pentes des courbes obtenues avec les solutions à différentes concentrations et la solution témoin, on détermine $C_{50}$, c'est-à-dire la concentration en anti-oxydant permettant l'inhibition de 50% de la peroxydation.

| | | | |
|---|---|---|---|
| Composé de l'exemple | 1 | : | $C_{50} = 2{,}70.10^{-6}$M |
| " " | 2 | : | $C_{50} = 1{,}22.10^{-6}$M |
| " " | 3 | : | $C_{50} = 1{,}10.10^{-6}$M |
| " " | 4 | : | $C_{50} = 0{,}70.10^{-6}$M |
| " " | 5 | : | $C_{50} = 0{,}35.10^{-6}$M |
| " " | 6 | : | $C_{50} = 0{,}90.10^{-6}$M |

Testé dans les mêmes conditions, le ditert.-butylhydroxy toluène (BHT), qui est un anti-oxydant classique, a une $C_{50}$ de $3{,}20.10^{-6}$ M.

EXEMPLES D'APPLICATION

Exemple A - Gel protecteur de la peau

| | | |
|---|---|---|
| - Composé de l'exemple 1 | | 0,12 g |
| - Glycérine | | 12,0 g |
| - Acide polyacrylique réticulé par un agent polyfonctionnel vendu sous la marque "CARBOPOL 934" par la Société GOODRICH | | 0,8 g |
| - Ethanol | | 15,0 g |
| - Conservateur | | 0,2 g |
| - Parfum | | 0,2 g |
| - Triéthanolamine | qs pH 5,3 | |
| - Eau déminéralisée | qsp | 100 g |

On dissout le composé de l'exemple 1 dans le mélange éthanolglycérine; on ajoute l'eau, le conservateur et le parfum. Dans cette phase aqueuse, on disperse le Carbopol de façon homogène et on ajuste le pH à 5,3 par la triéthanolamine.

Exemple B - Huile anti-solaire

On mélange les ingrédients suivants en chauffant éventuellement à 40-45°C pour homogénéiser :

| | | |
|---|---|---|
| - Composé de l'exemple 2 | | 0,6 g |
| - Benzoate d'alcools en $C_{12}$-$C_{15}$ vendu sous la marque "FINSOLV TN" (FINETEX) | | 30,0 g |
| - Huile de tournesol raffinée stabilisée | | 20,0 g |
| - Parfum | | 1,0 g |
| - Diméthyl polysiloxane cyclique vendu sous la marque "VOLATILE SILICONE 7207" (UNION CARBIDE) | qsp | 100 g |

Exemple C - Lait anti-solaire

| | | |
|---|---|---|
| - Composé de l'exemple 6 | | 0,25 g |
| - Benzylidène camphre | | 2,0 g |
| - Mélange d'esters d'acides gras, d'esters polyglycérolés et de tensio-actifs siliconés "ABIL WS08" (GOLDSCHMIDT) | | 5,0 g |
| - Vaseline blanche | | 2,0 g |
| - Cire d'abeilles | | 2,5 g |
| - Benzoate d'alcools en $C_{12}$-$C_{15}$ "FINSOLV TN" (FINETEX) | | 19,0 g |
| - Glycérine | | 5,0 g |
| - Chlorure de sodium | | 2,0 g |
| - Parfum | | 0,4 g |
| - Conservateur | | 0,2 g |
| - Eau déminéralisée | qsp | 100 g |

On dissout le composé de l'exemple 6 et le benzylidène camphre dans les corps gras et l'émulsionnant et on chauffe à 70-80°C; on chauffe à la même température la phase aqueuse constituée par l'eau, le chlorure de sodium et la glycérine; on ajoute sous vive agitation la phase aqueuse à la phase grasse, puis on laisse refroidir sous agitation modérée et vers 40°C, on ajoute parfum et conservateur. On obtient une émulsion eau-dans-l'huile.

Exemple D - Lait antisolaire

| | | |
|---|---|---|
| - Composé de l'exemple 1 | | 1,5 g |
| - p-méthoxycinnamate de 2-éthylhexyle "PARSOL MCX" (GIVAUDAN) | | 3,5 g |
| - 2-hydroxy-4-méthoxybenzophénone "UVINUL M40" | | 1,0 g |
| - Alcool cétylique | | 1,0 g |
| - Alcool oléocétylique à 30 moles d'oxyde d'éthylène "MERGITAL OC 30" (HENKEL) | | 5,0 g |
| - Alcool stéarylique | | 4,0 g |
| - Huile de synthèse de formule : $C_{15}H_{31}COOCH_2-\underset{OH}{\underset{\vert}{C}}H-CH_2OCH_2-\underset{C_2H_5}{\underset{\vert}{C}}H-C_4H_9$ | | 2,0 g |
| - Mélange 90/10 de 2-éthyl-hexanoate de cétostéaryle et de myristate d'isopropyle "CERAMOLL" (Créations Aromatiques) | | 2,0 g |
| - Huile de vaseline | | 8,0 g |
| - Propylène glycol | | 4,0 g |
| - Conservateur | | 0,2 g |
| - Parfum | | 0,4 g |
| - Eau déminéralisée | qsp | 100 g |

Il s'agit d'une émulsion huile-dans-l'eau. On dissout le composé de l'exemple 1 et les filtres dans les corps gras vers 70°-80°C; on chauffe à la même température la phase aqueuse constituée par l'eau, le propylène glycol et l'émulsionnant et on ajoute sous vive agitation la phase grasse à la phase aqueuse puis on laisse refroidir sous agitation modérée et on ajoute conservateur et parfum vers 40°C.

Exemple E - Stick antisolaire

On prépare le bâtonnet solide suivant :

| | | |
|---|---|---|
| - Composé de l'exemple 4 | | 1,0 g |
| - Cire d'Ozokérite "SP 1020" (STRAHL ET PITSCH) | | 20,0 g |
| - Cire d'abeilles | | 7,0 g |
| - Alcool oléique | | 12,0 g |
| - Lanoline hydrogénée "HYDROLAN H" (ONYX CHEMICAL) | | 8,0 g |
| - Huile de lanoline "ARGONOL 60" (WESTBROOK LANOLIN) | | 8,0 g |
| - Cire de carnauba | | 1,0 g |
| - Benzoate d'alcools en $C_{12}-C_{15}$ "FINSOLV TN" (FINETEX) | | 17,0 g |
| - Octaméthylcyclotétrasiloxane "ABIL K4" (GOLDSCHMIDT) | | 3,0 g |
| - Huile de vaseline | qsp | 100 g |

On fond les différents composés vers 70-75°C de façon à obtenir une phase liquide dans laquelle on dissout le composé de l'exemple 4. On coule cette solution dans des moules et on laisse refroidir.

Exemple F - Crème antisolaire

On prépare de la même façon que dans l'exemple D la crème suivante constituant une émulsion huile-dans-l'eau dont la phase aqueuse est constituée d'eau, de sorbitol, de lactate de sodium et de l'émulsionnant et dans laquelle on dissout le méthylsulfate de 4-[(2-oxo-3-bornylidène)méthyl] phényl triméthylammonium :

| | | |
|---|---|---|
| - Composé de l'exemple 5 | | 0,5 g |
| - Méthylsulfate de 4-[(2-oxo-3-bornylidène)méthyl]-phényl triméthylammonium | | 4,0 g |
| - Lactate de sodium à 60% | | 1,0 g |
| - Mélange d'alcool cétylstéarylique et d'alcool cétylstéarylique oxyéthyléné à 33 moles d'oxyde d'éthylène "SINNOWAX AO" (HENKEL) | | 7,5 g |
| - Mélange de mono et distéarate de glycérol non auto-émulsionnable | | 2,1 g |
| - Alcool cétylique | | 1,0 g |
| - Alcool myristique "SIPOL C14" (HENKEL) | | 0,6 g |
| - Sorbitol à 70% | | 3,0 g |
| - Palmitate d'isopropyle | | 10,0 g |
| - Huile de vaseline | | 7,0 g |
| - Conservateur | | 0,2 g |
| - Parfum | | 0,6 g |
| - Eau déminéralisée | qsp | 100 g |

Compositions pharmaceutiques utilisées par voie topique :

Exemple G - Onguent apaisant

(A appliquer sur une peau irritée pour calmer)

| | | |
|---|---|---|
| - Composé de l'exemple 1 | | 2,0 g |
| - Huile de vaseline fluide | | 9,1 g |
| - Silice vendue par la société DEGUSSA sous le nom de "AEROSIL 200" | | 9,2 g |
| - Myristate d'isopropyle | qsp | 100 g |

Exemple H - Crème (huile-dans-l'eau) anti-inflammatoire

| | | |
|---|---|---|
| - Composé de l'exemple 1 | | 3,0 g |
| - Dodécyl sulfate de sodium | | 0,8 g |
| - Glycérol | | 2,0 g |
| - Alcool stéarylique | | 20,0 g |
| - Triglycérides d'acides caprique/caprylique vendus par la société DYNAMIT NOBEL sous le nom de "MIGLYOL 812" | | 20,0 g |
| - Conservateurs | qs | |
| - Eau déminéralisée | qsp | 100 g |

Exemple I - Gel apaisant

| | | |
|---|---|---|
| - Composé de l'exemple 5 | | 1,0 g |
| - Hydroxypropyl cellulose vendue par la société HERCULES sous le nom de "KLUCEL HF" | | 2,0 g |
| - Ethanol | | 70,0 g |
| - Eau | qsp | 100 g |

**Revendications**

1. Dérivé de benzylidène camphre caractérisé par le fait qu'il répond à la formule :

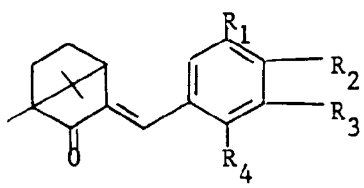

(I')

dans laquelle $R_1$ représente un atome d'hydrogène, un radical hydroxyle, un radical alkyle linéaire ou ramifié en $C_1$-$C_3$ ou un radical alcoxy en $C_1$-$C_8$ linéaire ou ramifié, $R_3$ représente un radical hydroxyle, un radical alkyle linéaire ou ramifié en $C_1$-$C_3$ ou un radical alcoxy linéaire ou ramifié en $C_1$-$C_8$, ou bien lorsque $R_1$ représente un atome d'hydrogène ou un radical hydroxyle, $R_3$ peut représenter un reste alkyle linéaire ou ramifié en $C_1$-$C_8$,

$R_2$ et $R_4$, identiques ou différents, représentent un atome d'hydrogène ou un radical hydroxyle, étant entendu que l'un au moins des symboles $R_2$ et $R_4$ représente un radical hydroxyle,

sous réserve que lorsque $R_1$ et $R_4$ représentent un atome d'hydrogène et $R_2$ un radical hydroxyle, $R_3$ ne représente pas un radical alcoxy.

2. Composé selon la revendication 1, caractérisé par le fait qu'il est choisi parmi le 3'-tert.-butyl-4'-hydroxy-3-benzylidène-camphre, le 3',5'-diméthyl-4'-hydroxy-3-benzylidène camphre, le 3', 5'-diisopropyl-4'-hydroxy-3-benzylidène camphre, le 3',5'-diméthoxy-4'-hydroxy-3-benzylidène camphre et le 3',4',5'-trihydroxy-3-benzylidène camphre.

3. Composition cosmétique, caractérisée par le fait qu'elle comprend dans un support cosmétiquement acceptable, une quantité efficace d'au moins un composé de formule (I') selon la revendication 1 ou 2, à

titre d'agent anti-oxydant et filtrant les rayons UV de longueurs d'onde comprises entre 280 et 380 nm.

4. Composition cosmétique selon la revendication 3, caractérisée par le fait qu'elle comprend à titre de composé (I'), au moins l'un des composés choisis parmi : le 3'-tert.-butyl-4'-hydroxy-3-benzylidène camphre, le 3'-5'-diméthyl-4'-hydroxy-3-benzylidène camphre, le 3',5'-diisopropyl-4'-hydroxy-3-benzylidène camphre, le 3',5'-diméthoxy-4'-hydroxy-3-benzylidène camphre et le 3',4',5'-trihydroxy-3-benzylidène camphre.

5. Composition cosmétique selon la revendication 3 ou 4, caractérisée par le fait qu'elle se présente sous forme d'émulsion, de lotion, de gel oléoalcoolique ou hydroalcoolique, de bâtonnet solide ou aérosol.

6. Composition cosmétique selon la revendication 5, caractérisée par le fait qu'elle contient en outre des adjuvants cosmétiques choisis parmi les épaississants, adoucissants, humectants, tensio-actifs, conservateurs, anti-mousses, parfums, huiles, cires, lanoline, monoalcools et polyols inférieurs, propulseurs, colorants et pigments.

7. Composition cosmétique selon la revendication 5 ou 6, caractérisée par le fait qu'elle constitue une composition protectrice de l'épiderme humain et contient 0,1 à 4% en poids de composé de formule (I').

8. Composition cosmétique selon la revendication 5 ou 6, se présentant sous forme de composition anti-solaire, caractérisée par le fait qu'elle contient 0,2 à 15% en poids de composé de formule (I').

9. Composition cosmétique anti-solaire selon la revendication 8, caractérisée par le fait qu'elle contient en outre un agent filtrant les rayons UV-B et/ou UV-A.

10. Composition cosmétique selon la revendication 3 ou 4, destinée à être appliquée sur les cheveux, caractérisée par le fait qu'elle se présente sous forme de shampooing, lotion, gel ou émulsion à rincer, lotion ou gel coiffants ou traitants, lotion ou gel pour le brushing ou la mise en plis, laque pour cheveux, composition de permanente, de décoloration ou de coloration et comprend 0,25 à 4% en poids de composé de formule (I').

11. Composition cosmétique selon la revendication 3 ou 4, se présentant sous forme d'une composition cosmétique colorée ou non, stabilisée à la lumière et à l'oxydation, caractérisée par le fait qu'elle est constituée par une composition capillaire, un produit de maquillage ou une composition pour les soins ou le traitement de la peau, comprenant 0,1 à 4% en poids de composé de formule (I').

12. Procédé de traitement cosmétique de la peau et des cheveux naturels ou sensibilisés pour les protéger contre le rayonnement ultraviolet, caractérisé par le fait qu'il consiste à appliquer sur la peau ou les cheveux une quantité efficace d'une composition cosmétique contenant au moins un dérivé de benzylidène camphre de formule (I') selon la revendication 1 ou 2.

13. Procédé de protection d'une composition cosmétique ou pharmaceutique contre les rayons ultra-violets et l'oxydation, caractérisé par le fait qu'il' consiste à incorporer à cette composition une quantité efficace d'au moins un composé de formule (I').

14. Utilisation en tant qu'agent anti-oxydant d'au moins un composé de formule (I) suivante :

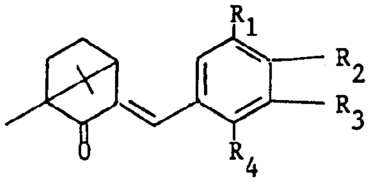

(I)

dans laquelle

$R_1$ représente un atome d'hydrogène, un radical hydroxyle, un radical alkyle linéaire ou ramifié en $C_1$-$C_3$ ou un radical alcoxy linéaire ou ramifié en $C_1$-$C_8$,

$R_3$ représente un radical hydroxyle, un radical alkyle linéaire ou ramifié en $C_1$-$C_3$ ou un radical alcoxy linéaire ou ramifié en $C_1$-$C_8$, ou bien lorsque $R_1$ représente un atome d'hydrogène ou un radical hy-

droxyle, $R_3$ peut représenter un reste alkyle linéaire ou ramifié en $C_1$-$C_8$,

$R_2$ et $R_4$, identiques ou différents, représentent un atome d'hydrogène ou un radical hydroxyle, étant entendu que l'un au moins des radicaux $R_2$ et $R_4$ représente un radical hydroxyle.

**15.** Utilisation en tant qu'agent anti-oxydant d'au moins un composé de formule (I) choisi parmi le 3'-tert.-butyl-4'-hydroxy-3-benzylidène camphre, le 3'-méthoxy-4'-hydroxy-3-benzylidène camphre, le 3'-éthoxy-4'-hydroxy-3-benzylidène camphre, le 3',5'-diméthyl-4'-hydroxy-3-benzylidène camphre, le 3',5'-diisopropyl-4'-hydroxy-3-benzylidène camphre, le 3'-5'-diméthoxy-4'-hydroxy-3-benzylidène camphre et le 3',4',5'-trihydroxy-3-benzylidène camphre.

**16.** Composé de formule (I) :

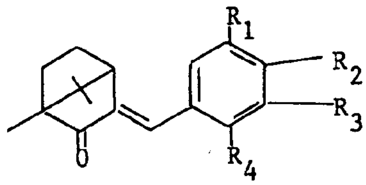

(I)

dans laquelle

$R_1$ représente un atome d'hydrogène, un radical hydroxyle, un radical alkyle linéaire ou ramifié en $C_1$-$C_3$ ou un radical alcoxy linéaire ou ramifié en $C_1$-$C_8$,

$R_3$ représente un radical hydroxyle, un radical alkyle linéaire ou ramifié en $C_1$-$C_3$ ou un radical alcoxy linéaire ou ramifié en $C_1$-$C_8$, ou bien lorsque $R_1$ représente un atome d'hydrogène ou un radical hydroxyle, $R_3$ peut représenter un reste alkyle linéaire ou ramifié en $C_1$-$C_8$,

$R_2$ et $R_4$, identiques ou différents, représentent un atome d'hydrogène ou un radical hydroxyle, étant entendu que l'un au moins des radicaux $R_2$ et $R_4$ représente un radical hydroxyle, pour son utilisation comme médicament.

**17.** Composé selon la revendication 16 caractérisé par le fait qu'il est choisi parmi le 3'-tert.-butyl-4'-hydroxy-3-benzylidène camphre, le 3'-méthoxy-4'-hydroxy-3-benzylidène camphre, le 3'-éthoxy-4'-hydroxy-3-benzylidène camphre, le 3',5'-diméthyl-4'-hydroxy-3-benzylidène camphre, le 3',5'-diisopropyl-4'-hydroxy-3-benzylidène camphre, le 3'-5'-diméthoxy-4'-hydroxy-3-benzylidène camphre et le 3',4',5'-trihydroxy-3-benzylidène camphre.

**18.** Composé de formule (I) selon la revendication 16 ou 17 pour son utilisation dans le traitement des inflammations et allergies cutanées.

**19.** Composition pharmaceutique, caractérisée par le fait qu'elle comprend une quantité efficace d'au moins un composé de formule (I) selon la revendication 16 ou 17, dans un support ou excipient non toxique.

**20.** Composition pharmaceutique destinée à être administrée par voie topique, se présentant sous forme de crème, onguent, pommade solution, gel, lotion, spray, suspension, caractérisée par le fait qu'elle contient au moins un composé de formule (I) selon la revendication 16 ou 17.

**21.** Composition pharmaceutique, destinée à être administrée par voie orale sous forme de comprimés, de gélules, de dragées, de sirops, de suspensions, de solutions, d'émulsions, caractérisée par le fait qu'elle contient au moins un composé de formule (I) selon la revendication 16 ou 17.

**22.** Composition pharmaceutique, selon la revendication 20, caractérisée par le fait qu'elle contient un composé actif de formule (I) dans des proportions comprises entre 0,25 et 4% en poids par rapport au poids total de la composition.

**23.** Utilisation d'un composé de formule (I) selon la revendication 16 pour la préparation d'un médicament destiné au traitement des inflammations et allergies cutanées.

**Patentansprüche**

1. Benzylidenkampferderivat, dadurch gekennzeichnet,daß es die Formel aufweist:

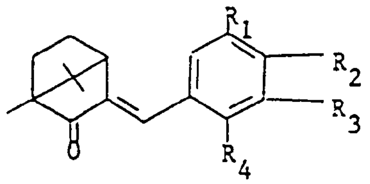

(I')

worin
$R_1$ ein Wasserstoffatom, einen Hydroxylrest, einen linearen oder verzweigten $C_{1-3}$-Alkylrest oder einen linearen oder verzweigten $C_{1-8}$-Alkoxyrest,
$R_3$ einen Hydroxylrest, einen linearen oder verzweigten $C_{1-3}$-Alkylrest oder einen linearen oder verzweigten $C_{1-8}$-Alkoxyrest, oder $R_3$ auch, wenn $R_1$ ein Wasserstoffatom oder einen Hydroxylrest darstellt, einen linearen oder verzweigten $C_{1-8}$-Alkylrest, und
$R_2$ und $R_4$ gleich oder verschieden, ein Wasserstoffatom oder einen Hydroxylrest darstellen, wobei mindestens einer der Reste $R_2$ und $R_4$ einen Hydroxylrest darstellt, mit der Maßgabe, daß, wenn $R_1$ und $R_2$ ein Wasserstoffatom und $R_2$ einen Hydroxylrest darstellen, $R_3$ keinen Alkoxyrest darstellt.

2. Verbindung gemäß Anspruch 1, dadurch gekennzeichnet, daß sie ausgewählt ist aus:
   - 3'-t-Butyl-4'-hydroxy-3-benzylidenkampfer,
   - 3',5'-Dimethyl-4'-hydroxy-3-benzylidenkampfer,
   - 3'-5'-Diisopropyl-4'-hydroxy-3-benzylidenkampfer,
   - 3',5'-Dimethoxy-4'-hydroxy-3-benzylidenkampfer,
   - 3'-4',5'-Trihydroxy-3-benzylidenkampfer.

3. Kosmetische Zusammensetzung, dadurch gekennzeichnet, daß sie in einer kosmetisch verträglichen Trägerunterlage eine wirksame Menge mindestens einer Verbindung der Formel (I') gemäß Anspruch 1 oder 2 als antioxidierendes und UV-Strahlen einer Wellenlänge von 280 bis 380 nm filterndes Mittel enthält.

4. Zusammensetzung gemäß Anspruch 3, dadurch gekennzeichnet, daß sie als Verbindung (I') mindestens eine der Verbindungen enthält, ausgewählt aus:
   - 3'-t-Butyl-4'-hydroxy-3-benzylidenkampfer,
   - 3',5'-Dimethyl-4'-hydroxy-3-benzylidenkampfer,
   - 3'-5'-Diisopropyl-4'-hydroxy-3-benzylidenkampfer,
   - 3',5'-Dimethoxy-4'-hydroxy-3-benzylidenkampfer,
   - 3'-4',5'-Trihydroxy-3-benzylidenkampfer.

5. Kosmetische Zusammensetzung gemäß Anspruch 3 oder 4, dadurch gekennzeichnet, daß sie in Form einer Emulsion, Lotion, eines oleoalkoholischen oder hydroalkoholischen Gels, Feststoffstäbchens oder Aerosols vorliegt.

6. Zusammensetzung gemäß Anspruch 5, dadurch gekennzeichnet, daß sie ausserdem kosmetische Hilfstoffe enthält, ausgewählt aus Verdickungs-, Weich-, Feuchtigkeits-, oberflächenaktiven, Konservierungs-, Antischaummitteln, Parfüms, Ölen, Wachsen, Lanolin, Niedrigmonoalkoholen und -polyolen, Treibmitteln, Färbemitteln und Pigmenten.

7. Kosmetische Zusammensetzung gemäß Anspruch 5 oder 6, dadurch gekennzeichnet, daß sie eine Zusammensetzung zum Schutz der menschlichen Haut darstellt und 0,1 bis 4 Gew.% Verbindung der Formel (I') enthält.

8. Kosmetische Zusammensetzung gemäß Anspruch 5 oder 6, die in Form einer Zusammensetzung zum Schutz vor schädlicher Sonneneinwirkung vorliegt, dadurch gekennzeichnet, daß sie 0,2 bis 15 Gew.% Verbindung der Formel (I') enthält.

9. Kosmetische Sonnenschutzzusammensetzung gemäß Anspruch 8, dadurch gekennzeichnet, daß sie ausserdem ein die UV-B- und/oder UV-A-Strahlen filterndes Mittel enthält.

10. Kosmetische Zusammensetzung gemäß Anspruch 3 oder 4 zur Aufbringung auf die Haare, dadurch gekennzeichnet, daß sie in Form eines Shampoos, einer Lotion, eines Gels oder einer Emulsion zur Spülung, einer Lotion oder eines Gels zur Frisur oder Behandlung, einer Lotion oder eines Gels zum Bürsten oder zur Formgebung, eines Lacks für die Haare, einer Zusammensetzung zur Dauerwelle, Entfärbung oder Färbung vorliegt und 0,25 bis 4 Gew.% Verbindung der Formel (I') enthält.

11. Kosmetische Zusammensetzung gemäß Anspruch 3 oder 4, die in Form einer gefärbten oder nicht gefärbten , gegen die Einwirkung von Licht und Oxidation stabilisierten kosmetischen Zusammensetzung vorliegt, dadurch gekennzeichnet, daß sie eine Zusammensetzung für die Haare, ein Schminkprodukt oder eine Zusammensetzung zur Pflege oder Behandlung der Haut darstellt, wobei sie 0,1 bis 4 Gew.% Verbindung der Formel (I') enthält.

12. Verfahren zur kosmetischen Behandlung der Haut und natürlicher oder sensibilisierter Haare zum Schutz vor UV-Strahlung, dadurch gekennzeichnet, daß man auf die Haut oder die Haare eine wirksame Menge einer kosmetischen Zusammensetzung aufbringt, die mindestens ein Benzylidenkampfer-Derivat der Formel (I') gemäß Anspruch 1 oder 2 enthält.

13. Verfahren zum Schutz einer kosmetischen oder pharmazeutischen Zusammensetzung vor UV-Strahlen und Oxidation, dadurch gekennzeichnet, daß man dieser Zusammensetzung eine wirksame Menge mindestens einer Verbindung der Formel (I') gemäß Anspruch 1 oder 2 zufügt.

14. Verwendung als Antioxidans mindestens einer Verbindung der folgenden Formel (I):

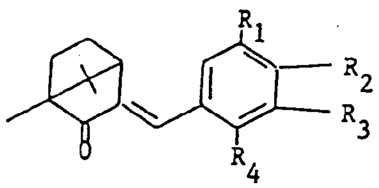

(I)

worin

$R_1$ ein Wasserstoffatom, einen Hydroxylrest, einen linearen oder verzweigten $C_{1-3}$-Alkylrest oder einen linearen oder verzweigten $C_{1-8}$-Alkoxyrest,

$R_3$ einen Hydroxylrest, einen linearen oder verzweigten $C_{1-3}$-Alkylrest oder einen linearen oder verzweigten $C_{1-8}$-Alkoxyrest, oder $R_3$ auch, wenn $R_1$ ein Wasserstoffatom oder einen Hydroxylrest darstellt, einen linearen oder verzweigten $C_{1-8}$-Alkylrest, und

$R_2$ und $R_4$ gleich oder verschieden, ein Wasserstoffatom oder einen Hydroxylrest darstellen, wobei mindestens einer der Reste $R_2$ und $R_4$ einen Hydroxylrest darstellt.

15. Verwendung als Antioxidans mindestens einer Verbindung der Formel (I), ausgewählt aus
- 3'-t-Butyl-4'-hydroxy-3-benzylidenkampfer,
- 3'-Methoxy-4'-hydroxy-3-benzylidenkampfer
- 3'-Ethoxy-4'-hydroxy-3-benzylidenkampfer,
- 3',5'-Dimethyl-4'-hydroxy-3-benzylidenkampfer,
- 3',5'-Diisopropyl-4'-hydroxy-3-benzylidenkampfer,
- 3',5'-Dimethoxy-4'-hydroxy-3-benzylidenkampfer,
- 3',4',5'-Trihydroxy-3-benzylidenkampfer.

16. Verbindung der Formel (I):

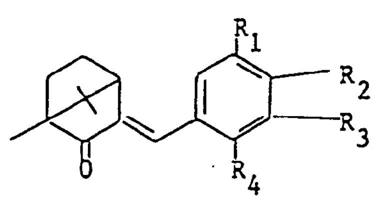

(I)

worin:
$R_1$ ein Wasserstoffatom, einen Hydroxylrest, einen linearen oder verzweigten $C_{1-3}$-Alkylrest oder einen linearen oder verzweigten $C_{1-8}$-Alkoxyrest,
$R_3$ einen Hydroxylrest, einen linearen oder verzweigten $C_{1-3}$-Alkylrest oder einen linearen oder verzweigten $C_1$-$_8$-Alkoxyrest, oder $R_3$ auch, wenn $R_1$ ein Wasserstoffatom oder einen Hydroxylrest darstellt, einen linearen oder verzweigten $C_{1-8}$-Alkylrest, und
$R_2$ und $R_4$, gleich oder verschieden, ein Wasserstoffatom oder einen Hydroxylrest darstellen, wobei mindestens einer der Reste $R_2$ und $R_4$ einen Hydroxylrest darstellt, zur Verwendung als Medikament.

**17.** Verbindung gemäß Anspruch 16, dadurch gekennzeichnet, daß sie ausgewählt ist aus:
- 3'-t-Butyl-4'-hydroxy-3-benzylidenkampfer,
- 3'-Methoxy-4'-hydroxy-3-benzylidenkampfer
- 3'-Ethoxy-4'-hydroxy-3-benzylidenkampfer,
- 3',5'-Dimethyl-4'-hydroxy-3-benzylidenkampfer,
- 3',5'-Diisopropyl-4'-hydroxy-3-benzylidenkampfer,
- 3',5'-Dimethoxy-4'-hydroxy-3-benzylidenkampfer, 3',4',5'-Trihydroxy-3-benzylidenkampfer.

**18.** Verbindung der Formel (1) gemäß Anspruch 16 oder 17 zur Verwendung bei der Behandlung von Entzündungen und Allergien der Haut.

**19.** Pharmazeutische Zusammensetzung, dadurch gekennzeichnet, daß sie eine wirksame Menge mindestens einer Verbindung der Formel (I) gemäß Anspruch 16 oder 17 in einem nichttoxischen Träger oder Exzipient enthält.

**20.** Pharmazeutische Zusammensetzung zur topischen Verabreichung, die in Form einer Creme, Salbe, Pommade, Lösung, eines Gels, einer Lotion, eines Sprays, einer Suspension vorliegt, dadurch gekennzeichnet, daß sie mindestens eine Verbindung der Formel (I) gemäß Anspruch 16 oder 17 enthält.

**21.** Pharmazeutische Zusammensetzung zur oralen Verabreichung in Form von Tabletten, Kapseln, Dragees, Sirups, Suspensionen, Lösungen, Emulsionen, dadurch gekennzeichnet, daß sie mindestens eine Verbindung der Formel (I) gemäß Anspruch 16 oder 17 enthält.

**22.** Pharmazeutische Zusammensetzung gemäß Anspruch 20, dadurch gekennzeichnet, daß sie die Wirkverbindung der Formel (I) in Mengenverhältnissen von 0,25 bis 4 Gew.%, bezogen auf Gesamtgewicht der Zusammenetzung, enthält.

**23.** Verwendung einer Verbindung der Formel (I) gemäß Anspruch 16 zur Herstellung eines Medikaments zur Behandlung von Entzündungen und Allergien der Haut.

## Claims

**1.** Derivative of benzylidenecamphor characterized in that it corresponds to the formula:

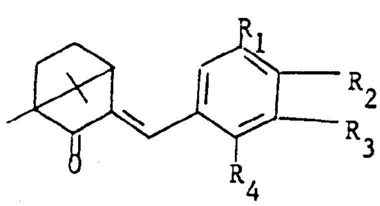

(I')

in which $R_1$ represents a hydrogen atom, a hydroxyl radical, a straight-chain or branched-chain $C_1$-$C_3$ alkyl radical, or a straight-chain or branched-chain $C_1$-$C_8$ alkoxy radical, $R_3$ represents a hydroxyl radical, a straight-chain or branched-chain $C_1$-$C_3$ alkyl radical, or a straight-chain or branched-chain $C_1$-$C_8$ alkoxy radical, or else when $R_1$ represents a hydrogen atom or a hydroxyl radical, $R_3$ can represent a straight-chain or branched-chain $C_1$-$C_8$ alkyl residue,

$R_2$ and $R_4$, identical or different, represent a hydrogen atom or a hydroxyl radical, it being understood that at least one of the symbols $R_2$ and $R_4$ represents a hydroxyl radical,

provided that when $R_1$ and $R_4$ represent a hydrogen atom and $R_2$ a hydroxyl radical, $R_3$ does not represent an alkoxy radical.

2. Compound according to Claim 1, characterized in that it is selected from 3'-tert-butyl-4'-hydroxy-3-benzylidenecamphor , 3',5'-dimethyl-4'-hydroxy-3-benzylidenecamphor, 3',5'-diisopropyl-4'-hydroxy-3-benzylidenecamphor, 3',5'-dimethoxy-4'-hydroxy-3-benzylidenecamphor, and 3',4',5'-trihydroxy-3-benzylidenecamphor.

3. Cosmetic composition, characterized in that it comprises, in a cosmetically acceptable vehicle, an effective amount of at least one compound of formula (I') according to Claim 1 or 2, as an antioxidant agent and screening out agent for UV rays of wavelengths comprised between 280 and 380 nm.

4. Cosmetic composition according to Claim 3, characterized in that it comprises, as the compound (I'), at least one of the compounds selected from: 3'-tert-butyl-4'-hydroxy-3-benzylidenecamphor, 3',5'-dimethyl-4'-hydroxy-3-benzylidenecamphor, 3',5'-diisopropyl-4'-hydroxy-3-benzylidenecamphor, 3',5'-dimethoxy-4-hydroxy-3-benzylidenecamphor, and 3',4',5'-trihydroxy-3-benzylidenecamphor.

5. Cosmetic composition according to Claim 3 or 4, characterized in that it takes the form of an emulsion, a lotion, an oleoalcoholic or aqueous-alcoholic gel, a solid stick, or an aerosol.

6. Cosmetic composition according to Claim 5, characterized in that it furthermore contains cosmetic adjuvants selected from thickeners, emollients, humectants, surface active agents, preservatives, antifoaming agents, perfumes, oils, waxes, lanoline, lower monohydric alcohols and polyols, propellants, colorants and pigments.

7. Cosmetic composition according to Claim 5 or 6, characterized in that it constitutes a composition protecting the human epidermis and contains 0.1 to 4% by weight of compound of formula (I').

8. Cosmetic composition according to Claim 5 or 6, which takes the form of an antisun composition, characterized in that it contains 0.2 to 15% by weight of compound of formula (I').

9. Antisun cosmetic composition according to Claim 8, characterized in that it furthermore contains an agent screening out UV-B and/or UV-A rays.

10. Cosmetic composition according to Claim 3 or 4, intended to be applied on the hair, characterized in that it takes the form of a shampoo, lotion, gel, or emulsion to be rinsed, styling or treating lotion or gel, lotion or gel for blow-drying or setting, hair lacquer, or a composition for permanent waving, bleaching or dyeing, and contains 0.25 to 4% by weight of compound of formula (I').

11. Cosmetic composition according to Claim 3 or 4, taking the form of a cosmetic composition, coloured or not coloured, stabilized to light and to oxidation, characterized in that it is constituted by a hair-care composition, a makeup product, or a composition for care or treatment of the skin, comprising 0.1 to 4% by weight of compound of formula (I').

12. Process for the cosmetic treatment of the skin and of hair, which is natural or has been sensitized in order to protect them against ultraviolet radiation , characterized in that it consists in applying to the skin or on the hair on an effective amount of a cosmetic composition containing at least one derivative of benzylidenecamphor of formula (I') according to Claim 1 or 2.

13. Process of protection of a cosmetic or pharmaceutical composition against ultraviolet rays and oxidation, characterized in that its consists in incorporating into this composition an effective amount of at least one compound of formula (I').

14. Utilization as an antioxidant agent of at least one compound of the following formula (I) :

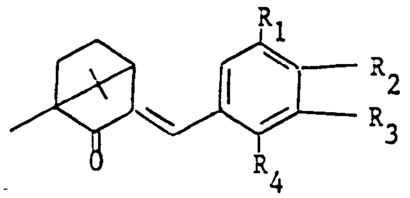

(I)

in which

$R_1$ represents a hydrogen atom, a hydroxyl radical, a straight-chain or branched-chain $C_1$-$C_3$ alkyl radical, or a straight-chain or branched-chain $C_1$-$C_8$ alkoxy radical,

$R_3$ represents a hydroxyl radical, a straight-chain or branched-chain $C_1$-$C_3$ alkyl radical, or a straight-chain or branched-chain $C_1$-$C_8$ alkoxy radical, or else when $R_1$ represents a hydrogen atom or a hydroxyl radical, $R_3$ can represent a straight-chain or branched-chain $C_1$-$C_8$ alkyl residue,

$R_2$ and $R_4$, identical or different, represent a hydrogen atom or a hydroxyl radical, it being understood that at least one of the radicals $R_2$ and $R_4$ represents a hydroxyl radical.

**15.** Utilization as an antioxidant agent of at least one compound of formula (I) selected from 3'-tert-butyl-4'-hydroxy-3-benzylidenecamphor, 3'-methoxy-4'-hydroxy-3-benzylidenecamphor, 3'-ethoxy-4'-hydroxy-3-benzylidene camphor, 3',5'-dimethyl-4'-hydroxy-3-benzylidenecamphor, 3',5'-diisopropyl-4'-hydroxy-3-benzylidenecamphor, 3',5'-dimethoxy-4'-hydroxy-3-benzylidenecamphor, end 3',4',5'-trihydroxy-3-benzylidenecamphor.

**16.** Compound of formula (I):

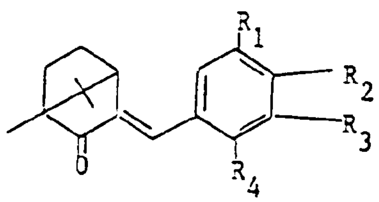

(I)

in which

$R_1$ represents a hydrogen atom, a hydroxyl radical, a straight-chain or branched-chain $C_1$-$C_3$ alkyl radical, or a straight-chain or branched-chain $C_1$-$C_8$ alkoxy radical,

$R_3$ represents a hydroxyl radical, a straight-chain or branched-chain $C_1$-$C_3$ alkyl radical, or a straight-chain or branched chain $C_1$-$C_8$ alkoxy radical, or else when $R_1$ represents a hydrogen atom or a hydroxyl radical, $R_3$ can represent a straight-chain or branched-chain $C_1$-$C_8$ alkyl residue,

$R_2$ and $R_4$, identical or different, represent a hydrogen atom or a hydroxyl radical, it being understood that at least one of the radicals $R_2$ and $R_4$ represents a hydroxyl radical,

for its utilization as a medicament.

**17.** Compound of formula (I) according to Claim 16, characterized in that it is selected from 3'-tert-butyl-4'-hydroxy-3-benzylidenecamphor, 3'-methoxy-4'-hydroxy-3-bensylidenecamphor, 3'-ethoxy-4'-hydroxy-3-bensylidenecamphor, 3',5'-dimethyl-4'-hydroxy-3-bensylidenecamphor, 3',5'-disopropyl-4'-hydroxy-3-bensylidenecamphor, 3',5'-dimethoxy-4'-hydroxy-3-bensylidenecamphor, and 3',4',5'-trihydroxy-3-bensylidenecamphor.

**18.** Compound of Formula (I) according to Claim 16 or 17 for its utilization in the treatment of cutaneous inflammations and allergies.

**19.** Pharmaceutical composition, characterized in that it comprises an effective amount of at least one compound of formula (I) according to Claim 16 or 17, in a non-toxic vehicle or excipient.

**20.** Pharmaceutical composition intended to be applied topically, taking the form of a cream, ointment, pomade, solution, gel, lotion, spray or suspension, characterized in that it contains at least one compound of formula (I) according to Claim 16 or 17.

21. Pharmaceutical composition intended to be administered orally in the form of tablets, hard gelatin capsules, sugar-coated pills, syrups, suspensions, solutions or emulsions, characterized in that it contains at least one compound of formula (I) according to Claim 16 or 17.

22. Pharmaceutical composition according to Claim 20, characterized in that it contains the active compound of formula (I) in proportions comprised between 0.25 and 4% by weight with respect to the total weight of the composition.

23. Utilization of a compound of formula (I) according to Claim 16 for the preparation of a medicament intended for the treatment of cutaneous inflammations and allergies.